# EUROPEAN PATENT SPECIFICATION

(11) **EP 4 727 498 B1**
(45) Date of publication and mention of the grant of the patent: **30.09.2026**
(21) Application number: 23739727.8
(22) Date of filing: 13.06.2023
(51) Int. Cl.: A61F 5/44, A61F 5/451

(54) **AT LEAST ONE POROUS MATERIAL ATTACHED TO A FLUID IMPERMEABLE BARRIER**
MINDESTENS EIN PORÖSES MATERIAL BEFESTIGT AN EINER FLÜSSIGKEITSUNDURCHLÄSSIGEN BARRIERE
AU MOINS UN MATÉRIAU POREUX FIXÉ À UNE BARRIÈRE IMPERMÉABLE AUX FLUIDES

(43) Date of publication of application: 22.04.2026
(73) Proprietor: Purewick Corporation, Covington, Georgia 30014-1498 (US)
(72) Inventor: NEWTON, Camille Rose, Bonsall, California 92003 (US)
(74) Representative: Studio Torta S.p.A.
(86) International application number: PCT/US2023/025192
(87) International publication number: WO 2024/258400

(56) References cited:
- WO-A1-2023/038950
- AU-A1- 2021 299 304

## Description

### BACKGROUND

A person or animal may have limited or impaired mobility so typical urination processes are challenging or impossible. For example, a person may experience or have a disability that impairs mobility. A person may have restricted travel conditions such as those experienced by pilots, drivers, and workers in hazardous areas. Additionally, sometimes bodily fluids collection is needed for monitoring purposes or clinical testing.

An example of a fluid collection assembly is disclosed in document AU 2021299304A1.

Urinary catheters, such as a Foley catheter, can address some of these circumstances, such as incontinence. Unfortunately, urinary catheters can be uncomfortable, painful, and can lead to complications, such as infections. Additionally, bed pans, which are receptacles used for the toileting of bedridden individuals are sometimes used. However, bedpans can be prone to discomfort, spills, and other hygiene issues.

There is therefore a substantial need for improved fluid collection devices.

### SUMMARY

The aforementioned aims are reached by a fluid collection assembly, a fluid collection system and a method of forming a fluid collection assembly as as claimed in the appended set of claims.

Embodiments disclosed herein are related to fluid collection assemblies including at least one porous material attached to the fluid impermeable barrier, fluid collection assemblies including the same, and methods of making and using the same.

Features from any of the disclosed embodiments may be used in combination with one another, without departing from the scope of the appended set of claims. In addition, other features and advantages of the present disclosure will become apparent to those of ordinary skill in the art through consideration of the following detailed description and the accompanying drawings.

### BRIEF DESCRIPTION OF THE DRAWINGS

The drawings illustrate several embodiments of the present disclosure, wherein identical reference numerals refer to identical or similar elements or features in different views or embodiments shown in the drawings.
**FIGS. 1A** and **1B** are isometric top and bottom views, respectively, of a fluid collection assembly, according to an embodiment.
**FIGS. 1C** and **1D** are cross-sectional views of the fluid collection assembly taken along planes 1C-1C and 1D-1D, respectively.
**FIG. 1E** is an enlarged cross-sectional view of the fluid collection assembly taken from circle 1E of **FIG. 1C** before the porous material is attached to the top and bottom panels.
**FIG. 1F** is an enlarged cross-sectional view of the fluid collection assembly taken from circle 1E of **FIG. 1C** after the porous material is attached to the top and bottom panels.
**FIG. 2A** is a cross-sectional view of a method of forming a fluid collection assembly, according to an embodiment.
**FIG. 2B** is a cross-sectional view of the fluid collection assembly after being formed.
**FIGS. 3** and **4** are top isometric views of different fluid collection assemblies having a porous material seal extending along only a portion of an outer periphery of the porous material, according to different embodiments.
**FIG. 5A** is a bottom plan view of a fluid collection assembly, according to an embodiment.
**FIG. 5B** is a cross-sectional view of the fluid collection assembly taken along plane 5B-5B shown in **FIG. 5A****.**
**FIG. 6** is a block diagram of a fluid collection system for fluid collection, according to an embodiment.

### DETAILED DESCRIPTION

Embodiments disclosed herein are related to fluid collection assemblies including at least one porous material attached to the fluid impermeable barrier, fluid collection assemblies including the same, and methods of making and using the same. An example fluid collection assembly includes a fluid impermeable barrier. The fluid impermeable barrier defines at least a chamber, at least one opening, and a fluid outlet. The fluid impermeable barrier also includes a top panel and a bottom panel opposite the top panel. The fluid collection assembly includes at least one porous material disposed in the chamber. The at least one porous material includes a top end region and a bottom end region. At least the edges of the bottom end region of the porous material is fixedly attached to the top panel and the bottom panel.

During use, the fluid collection assembly may be positioned on a patient *(i.e.,* the individual using the fluid collection assembly) such that the opening is positioned adjacent to a urethral opening (e.g., female urethral opening or a buried penis) of the patient or have the penis of the patient positioned through the opening and into the chamber. The patient may discharge one or more bodily fluids (e.g., urine, blood, sweat, etc.). The bodily fluids discharged by the patient may enter the chamber and be received into the porous material. The bodily fluids received into the porous material may flow towards the fluid outlet or the inlet of the conduit disposed in the chamber. For example, a suction may be provided to the chamber with a vacuum source that is in fluid communication with the chamber. The suction may cause the bodily fluids to preferentially flow towards the fluid outlet or the inlet of the conduit. The suction may then remove the bodily fluids from the chamber.

The fluid collection assemblies disclosed herein that include the porous material attached to the panels of the fluid impermeable barrier are an improvement over conventional fluid collection assemblies. For example, some conventional fluid collection assemblies include a porous material disposed in a chamber. In some conventional fluid collection assemblies, the porous material is merely disposed in the chamber without actually attaching the porous material to the fluid impermeable barrier. In such conventional fluid collection assemblies, the porous material may be maintained in the chamber by having the porous material press against the fluid impermeable barrier and/or selecting the porous material to exhibit a size that is greater than an opening formed in the fluid impermeable barrier. In other conventional fluid collection assemblies, one to a few holes may be formed in the porous material and portions of the fluid impermeable barrier adjacent to the holes may be attached together. In other conventional fluid collection assemblies, the porous material is physically attached to one side of the fluid impermeable barrier but not the other side to simplify manufacturing of such fluid collection assemblies and prevent adhesives or other attachments from blocking or otherwise obstructing the porous through the porous material through which the bodily fluids flow.

The conventional fluid collection assemblies disclosed above may have gaps forms between the porous material and the fluid impermeable barrier. When a suction is provided to the chamber of such fluid collection assemblies, the suction provided to the chambers from a vacuum source preferably removes air from such gaps instead of the bodily fluids held in the porous material. Sometimes, the removal of the air from the gaps causes the fluid impermeable barrier to be suctioned against the porous material thereby removing the gaps and promoting the flow of bodily fluids out of the porous material. Other times, the removal of the air from the gaps at least one of merely pulls more air into the gaps thereby preventing the fluid impermeable barrier from being suction against the porous material. The removal of the air from the gaps may also form folds or wrinkles in the fluid impermeable barrier when the fluid impermeable barrier is suctioned against the porous material through which the suction preferentially pulls air instead of bodily fluids from the chamber. The removal of the air from the gaps may further delay the suctioning of the fluid impermeable barrier against the porous material. Any of these above issues may prevent or inhibit removal of the bodily fluids from the porous material and from the chamber. These issues are especially significant with conventional male fluid collection assemblies that include thin and flimsy fluid impermeable barriers and/or are configured to lie flat when the conventional fluid collection assembly is disposed on a flat surface.

Another issues associated with at least some of the above-discussed conventional fluid collection assemblies is that the porous materials are not maintained against the fluid outlet or the inlet of the conduit. For example, the porous materials of at least some of the above discussed fluid collection assemblies may contact the fluid outlet or the inlet of the conduit of the conventional fluid collection assemblies prior to use. However, movement of the fluid collection assembly during use may cause the porous material to bend, be pulled, or otherwise caused to move away from the fluid outlet or the inlet of the conduit. Such movement may cause the formation of gaps between the porous material and the inlet of the fluid outlet or the inlet of the conduit through which the suction provided to the chamber removes air instead of bodily fluids. Also, moving the porous material away from the fluid outlet or the inlet of the conduit may cause the suction to pull the fluid impermeable barrier towards the fluid outlet or the inlet of the conduit, especially when the fluid impermeable barrier is formed from thin and flimsy material or the fluid collection assembly is configured to lie flat on a flat surface. Pulling the fluid impermeable barrier towards the fluid outlet or the inlet of the conduit may cause the fluid impermeable barrier to at least partially block the fluid outlet or the inlet of the conduit thereby preventing or at least inhibiting removal of the bodily fluids from the chamber.

The fluid collection assemblies disclosed herein that include the porous material attached to the panels of the fluid impermeable barrier are an improvement over conventional fluid collection assemblies since the fluid collection assemblies disclosed herein prevent or at least inhibit the formation of gaps. For example, the fluid collection assemblies disclosed herein include at least a portion of the porous material attached to both sides of the fluid impermeable barrier thereabout. Such attachments hold the fluid impermeable barrier against the porous material thereby preventing the formation of gaps. Such attachments may also maintain the porous material against the fluid outlet or the inlet of the conduit which, in turn, prevents the fluid impermeable barrier from moving towards the fluid outlet or the inlet of the conduit.

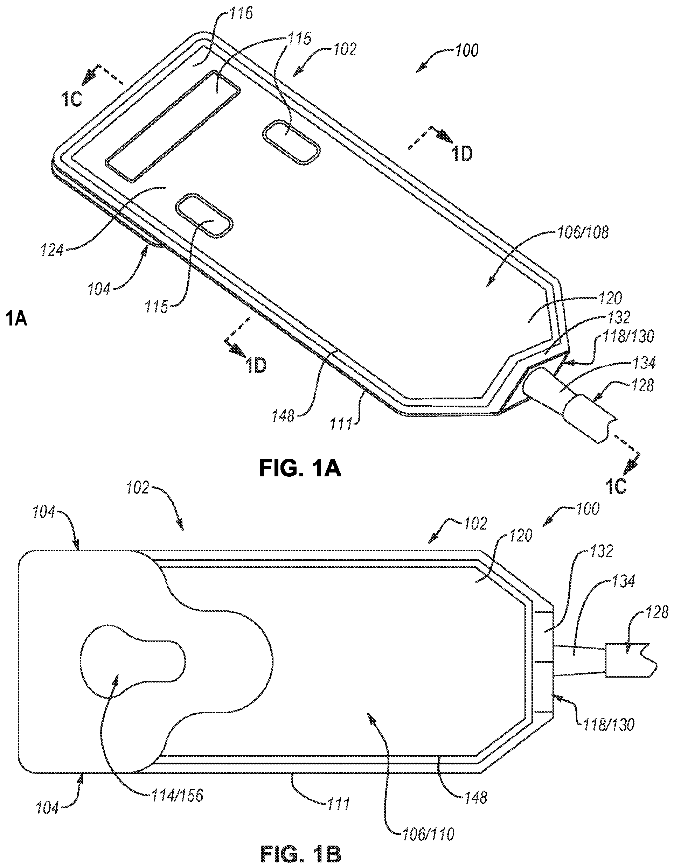

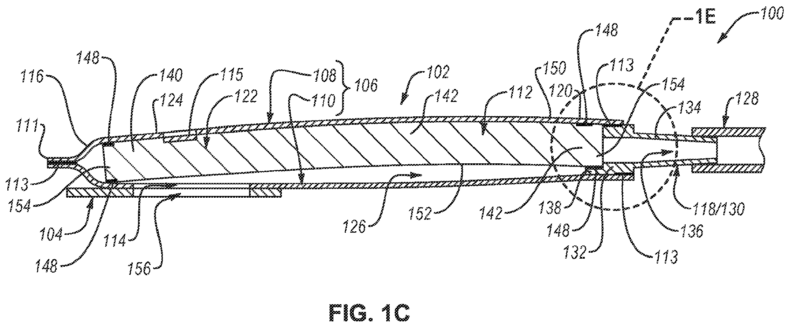

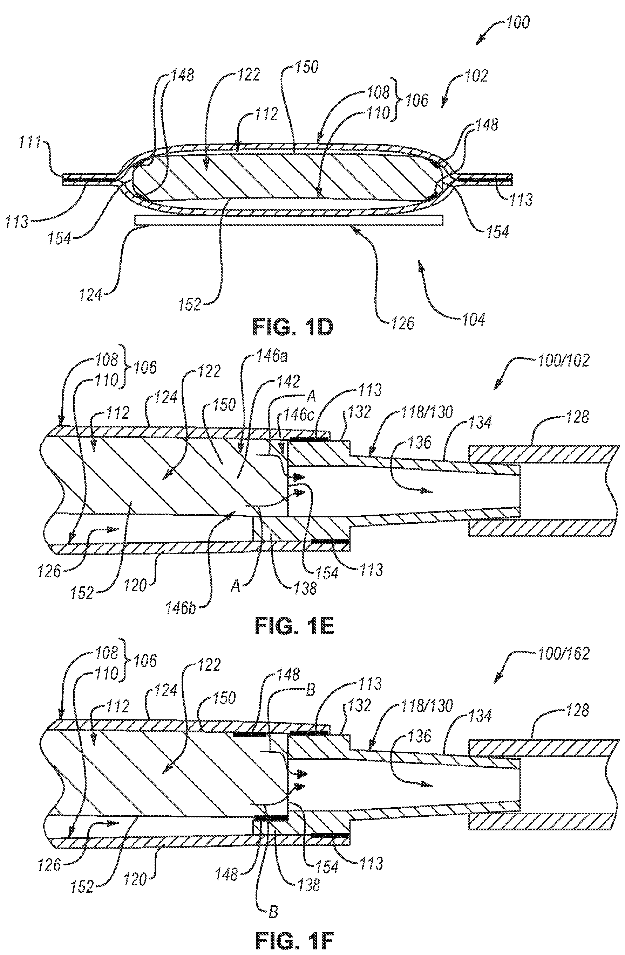

**FIGS. 1A** and **1B** are isometric top and bottom views, respectively, of a fluid collection assembly 100, according to an embodiment. **FIGS. 1C** and **1****D** are cross-sectional views of the fluid collection assembly 100 taken along planes 1C-1C and 1D-1D, respectively. The fluid collection assembly 100 is an example of a male fluid collection assembly though, in some embodiments, the fluid collection assembly 100 may be used to receive bodily fluids from a female urethral opening. The fluid collection assembly 100 includes a sheath 102 and a base 104. The base 104 is configured to be attached (e.g., permanently attached to or configured to be permanently attached) to the sheath 102. The base 104 is also configured to be attached to the region about the urethral opening (e.g., penis) of the individual.

The sheath 102 includes a fluid impermeable barrier 106 that is at least partially formed from a top panel 108 and a bottom panel 110. The fluid impermeable barrier 106 also defines a chamber 112 between the top panel 108 and the bottom panel 110, an opening 114 at a proximal end region 116 of the sheath 102, and a fluid outlet 118 at a distal end region 120 of the sheath 102. The sheath 102 also includes at least one porous material 122 disposed in the chamber 112.

The top panel 108 and the bottom panel 110 may be attached or integrally formed together (e.g., exhibits single piece construction). In an embodiment, as illustrated, the top panel 108 and the bottom panel 110 are distinct sheets. In such an embodiment, the top and bottom panels 108, 110 may be attached together along the edges 111 using one or more panel seals 113 (shown using bolded lines in **FIGS. 1C-1F****).** The panels seals 113 may include an ultrasonic ("US") weld, a radio frequency ("RF") weld, an adhesive, or any other suitable attachment that attaches the top and bottom panels 108, 112 together in a manner that prevents or at least inhibits flow of the bodily fluids between the top and bottom panels 108, 112.

The inner surface(s) of the fluid impermeable barrier 106 (e.g., inner surfaces of the top and bottom panels 108, 110 at least partially defines the chamber 112 within the fluid collection assembly 100. The fluid impermeable barrier 106 temporarily stores the bodily fluids in the chamber 112. The fluid impermeable barrier 106 substantially prevents the bodily fluids from passing through the fluid impermeable barrier 106.

The fluid impermeable barrier 106 may be formed of any suitable fluid impermeable material(s), such as a fluid impermeable polymer (e.g., silicone, polypropylene, polyethylene, polyethylene terephthalate, neoprene, a polycarbonate, etc.), a metal film, natural rubber, another suitable material, any other fluid impermeable material disclosed herein, or combinations thereof. As such, the fluid impermeable barrier 106 substantially prevents the bodily fluids from passing through the fluid impermeable barrier 106. In an example, the fluid impermeable barrier 106 may be air permeable and fluid impermeable. In such an example, the fluid impermeable barrier 106 may be formed of a hydrophobic material that defines a plurality of pores. At least one or more portions of at least an outer surface 124 of the fluid impermeable barrier 106 may be formed from a soft and/or smooth material, thereby reducing chaffing.

In an embodiment, at least one of the top panel 108 or the bottom panel 110 is formed from an at least partially transparent fluid impermeable material, such as polyethylene, polypropylene, polycarbonate, or polyvinyl chloride. Forming at least one of the top panel 108 or the bottom panel 110 from an at least partially transparent fluid impermeable material allows a person (e.g., medical practitioner) to examiner the penis. In some embodiments, both the top panel 108 and the bottom panel 110 are formed from at least partially transparent fluid impermeable material. Selecting at least one of the top panel 108 or the bottom panel 110 to be formed from an at least partially transparent impermeable material allows the penis to be examined without detaching the entire fluid collection assembly 100 from the region about the penis. For example, the chamber 112 may include a penis receiving area 126 that is configured to receive the penis of the individual when the penis extends into the chamber 112. The penis receiving area 126 may be defined by at least the porous material 122 and at least a portion of the at least partially transparent material of the top panel 108 and/or the bottom panel 110. In other words, the porous material 122 is positioned in the chamber 112 such that the porous material 122 is not positioned between the penis and at least a portion of the transparent portion of the top panel 108 and/or bottom panel 110 when the penis is inserted into the chamber 112 through the opening 114. The porous material 122 is generally not transparent and, thus, the portion of the at least partially transparent material of the top panel 108 and/or the bottom panel 110 that defines the penis receiving area 126 forms a window which allows the person to view into the penis receiving area 126 and examine the penis.

In an embodiment, the fluid impermeable barrier 106 may include one or more vents 115. The vents 115 may allow air to enter the chamber 112 and flow towards to the fluid outlet 118. Such air flow may promote the flow of the bodily fluids towards the fluid outlet 118. The vents 115 may include an air-permeable and water-impermeable filter to prevent the bodily fluids from leaking out of the chamber 112 via the vents 115.

The opening 114 defined by the fluid impermeable barrier 106 provides an ingress route for bodily fluids to enter the chamber 112 when the penis is a buried penis and allow the penis to enter the chamber 112 (e.g., the penis receiving area 126) when the penis is not buried. The opening 114 may be defined by the fluid impermeable barrier 106 (e.g., an inner edge of the fluid impermeable barrier 106). For example, the opening 114 is formed in and extends through the fluid impermeable barrier 106 thereby enabling bodily fluids to enter the chamber 112 from outside of the fluid collection assembly 100.

The fluid impermeable barrier 106 defines the fluid outlet 118 sized to receive the conduit 128. The conduit 128 may be at least partially disposed in the chamber 112 or otherwise in fluid communication with the chamber 112 through the fluid outlet 118. The fluid outlet 118 may be sized and shaped to form an at least substantially fluid tight seal against the conduit 128 thereby substantially preventing the bodily fluids from escaping the chamber 112.

In an embodiment, the fluid impermeable barrier 106 includes a cap 130 that forms the fluid outlet 118. The cap 130 exhibits a rigidity that is greater than the rigidity of the top and bottom panels 108, 110. The increased rigidity of the cap 130 facilitates attachment of the conduit 128 to the fluid outlet 118 *(e.g.,* by interference fit) than if the conduit 128 was attached directly to the top and bottom panels 108, 112. The cap 130 may include a connection portion 132 that is configured to be attached to the top and bottom panels 108, 110. The cap 130 may also include a conduit portion 134 extending from the connection portion 132 that is configured to be attached to the conduit 128. The cap 130 defines a passageway 136 extending through the connection portion 132 and the conduit portion 134 that allows the body fluids to flow through the cap 130 such that the bodily fluids may be removed from the chamber 112 into the conduit 128. The cap 130 may include a flange 138 extending from the connection portion 132 into the chamber 112. The flange 138 may extend adjacent to the bottom panel 110. The flange 138 may provide a location where the porous material 122 may be attached to the cap 130 via the porous material seal 148.

In an embodiment, the fluid outlet 118 may be formed from a portion of the top panel 108 and the bottom panel 110 that are not attached or integrally formed together. In such an embodiment, the fluid impermeable barrier 106 may not include the cap 130 which may facilitate manufacturing of the fluid collection assembly 100 may decreasing the number of parts that are used to form the fluid collection assembly 100 and may decrease the time required to manufacture the fluid collection assembly 100. The lack of the cap 130 may make securing the conduit 128 to the fluid outlet 118 using interference fit to be difficult though, it is noted, attaching the conduit 128 to the fluid outlet 118 may still be possible. As such, the conduit 128 may be attached to the fluid outlet 118 (e.g., to the top and bottom panels 108, 110) using an adhesive, a weld, or otherwise bonding the fluid outlet 118 to the fluid outlet 118. Attaching the conduit 128 to the fluid outlet 118 may prevent leaks and may prevent the conduit 128 from inadvertently becoming detached from the fluid outlet 118. In an example, the conduit 128 may be attached to the fluid outlet 118 in the same manufacturing step that attaches the top and bottom panels 108, 110 together.

As previously discussed, the sheath 102 includes at least one porous material 122 disposed in the chamber 112. The porous material 122 may direct the bodily fluids to one or more selected regions of the chamber 112, such as away from the penis and towards the fluid outlet 118. In an example, the porous material 122 may be formed from a single layer (as shown), two layers (e.g., a fluid permeable outer layer extending across the opening 114 and a fluid permeable inner layer since the fluid permeable outer layer may be formed from a relatively foldable, flimsy, or otherwise easily deformable material), or three or more layers. If the porous material 122 includes a plurality of layers, the plurality of layers may be attached together using an adhesive, entanglement between the fibers of the different layers, or any other suitable technique. Attaching the plurality of layers of the porous material 122 together prevents the formation of gaps between the layers through which the suction provided to the chamber 112 may pull air instead of bodily fluids. In an example, the porous material 122 may be formed from a nonwoven material or a woven material (e.g., spun nylon fibers). In an example, the porous material 122 may include at least one material exhibiting substantially no absorption or at least one absorbent or adsorbent material.

In an embodiment, the porous material 122 may be a sheet that is generally planar. Forming the porous material 122 as a sheet may facilitate the manufacturing of the fluid collection assembly 100. For example, forming the porous material 122 as a sheet allows the top panel 108, the bottom panel 110, and the porous material 122 to each be sheets. During the manufacturing of the fluid collection assembly 100, the top panel 108, the bottom panel 110, and the porous material 122 may be stacked and then attached to each other in the same manufacturing step. For instance, the porous material 122 may exhibit a shape that is the same size or, more preferably, slightly smaller than the size of the top panel 108 and the bottom panel 110. As such, attaching the top panel 108 and the bottom panel 110 together along the outer edges thereof may also attach the porous material 122 to the top panel 108 and the bottom panel 110. The porous material 122 may be slightly smaller than the top panel 108 and the bottom panel 110 such that the top panel 108 and/or the bottom panel 110 extend around the porous material 122 such that the porous material 122 does not form a passageway through the fluid impermeable barrier 106 through which the bodily fluids may leak. Also, attaching the porous material 122 to the top panel 108 and/or the bottom panel 110 may prevent the porous material 122 from significantly moving in the chamber 112, such as preventing the porous material 122 from bunching together near the fluid outlet 118. In an example, the porous material 122 may be attached to the top panel 108 or the bottom panel 110 (e.g., via an adhesive) before or after attaching the top panel 108 to the bottom panel 110. In an example, the porous material 122 may merely be disposed in the chamber 112 without attaching the porous material 122 to at least one of the top panel 108 or the bottom panel 110. In an embodiment, the porous material 122 may exhibit shapes other than a sheet, such as a hollow generally cylindrical shape.

In an embodiment, the porous material 122 may be configured to wick any bodily fluids away from the opening 114, thereby preventing the bodily fluids from escaping the chamber 112. The permeable properties referred to herein may be wicking, capillary action, diffusion, or other similar properties or processes, and are referred to herein as "permeable" and/or "wicking." Such "wicking" and/or "permeable" properties may not include absorption of the bodily fluids into at least a portion of the porous material 122, such as not include adsorption of the bodily fluids into the fluid permeable inner layer. Put another way, substantially no absorption or solubility of the bodily fluids into the material may take place after the material is exposed to the bodily fluids and removed from the bodily fluids for a time. While no absorption or solubility is desired, the term "substantially no absorption" may allow for nominal amounts of absorption and/or solubility of the bodily fluids into the porous material 122 (e.g., absorbency), such as less than about 30 wt% of the dry weight of the porous material 122, less than about 20 wt%, less than about 10 wt%, less than about 7 wt%, less than about 5 wt%, less than about 3 wt%, less than about 2 wt%, less than about 1 wt%, or less than about 0.5 wt% of the dry weight of the porous material 122. The porous material 122 may also wick the bodily fluids generally towards an interior of the chamber 112, as discussed in more detail below. In an embodiment, the porous material 122 may include at least one absorbent or adsorbent material.

In an embodiment, the porous material 122 may include the fluid permeable outer layer disposed in the chamber 112. The fluid permeable outer layer may cover at least a portion (e.g., all) of the opening 114. The fluid permeable outer layer may be composed to wick the bodily fluids away from the opening 114, thereby preventing the bodily fluids from escaping the chamber 112. In an embodiment, the fluid permeable outer layer may include any material that may wick the bodily fluids. For example, the fluid permeable outer layer may include fabric, such as a gauze (e.g., a silk, linen, or cotton gauze), another soft fabric, another smooth fabric, a nonwoven material (e.g., a vertically lapped nonwoven material), or any of the other porous materials disclosed herein. Forming the fluid permeable outer layer from gauze, soft fabric, and/or smooth fabric may reduce chaffing caused by the fluid collection assembly 100.

The fluid collection assembly 100 may include the fluid permeable inner layer disposed in the chamber 112. The fluid permeable inner layer is configured to support the fluid permeable outer layer since the fluid permeable outer layer may be formed from a relatively foldable, flimsy, or otherwise easily deformable material. For example, the fluid permeable inner layer may be positioned such that the fluid permeable outer layer is disposed between the fluid permeable inner layer and the fluid impermeable barrier 106. As such, the fluid permeable inner layer may support and maintain the position of the fluid permeable outer layer. The fluid permeable inner layer may include any material that may wick, absorb, adsorb, or otherwise allow fluid transport of the bodily fluids, such as any of the fluid permeable outer layer materials disclosed herein above. For example, the fluid permeable outer layer material(s) may be utilized in a more dense or rigid form than in the fluid permeable outer layer when used as the fluid permeable inner layer. The fluid permeable inner layer may be formed from any fluid permeable material that is less deformable than the fluid permeable outer layer. For example, the fluid permeable inner layer may include a porous polymer (e.g., nylon, polyester, polyurethane, polyethylene, polypropylene, etc.) structure or an open cell foam, such as spun nylon fiber. In some examples, the fluid permeable inner layer may include a nonwoven material, such as a vertically lapped nonwoven material. In some examples, the fluid permeable inner layer may be formed from a natural material, such as cotton, wool, silk, or combinations thereof. In such examples, the material may have a coating to prevent or limit absorption of fluid into the material, such as a water repellent coating. In some examples, the fluid permeable inner layer may be formed from fabric, felt, gauze, or combinations thereof.

In some examples, the fluid permeable outer layer may be optional. For example, the porous material 122 may include only the fluid permeable inner layer. In some examples, the fluid permeable inner layer may be optionally omitted from the fluid collection assembly 100. For example, the porous material 122 may only include the fluid permeable outer layer. Other examples of porous materials that may be included in the fluid collection assembly 100 are disclosed in PCT Patent Application No. PCT/US2021/039866 filed on June 30, 2021, PCT International Application No. PCT/US2022/011281 filed on January 5, 2022, PCT International Application No. PCT/US2022/042719 filed on September 7, 2022, PCT International Application No. PCT/US2022/042725 filed on September 7, 2022, U.S. Provisional Patent Application No. 63/241,564 filed on September 8, 2021, PCT International Application No. PCT/US2022/015418 filed on February 7, 2022, and PCT International Application No. PCT/US2022/015420 filed on February 7, 2022.

In an embodiment, at least a portion of the porous material 122 (e.g., one or more of the fluid permeable outer layer or, more particularly, the fluid permeable inner layer) may be hydrophobic. The porous material 122 may be hydrophobic when the porous material 122 exhibits a contact angle with water (a major constituent of bodily fluids) that is greater than about 90°, such as in ranges of about 90° to about 120°, about 105° to about 135°, about 120° to about 150°, about 135° to about 175°, or about 150° to about 180°. The hydrophobicity of the porous material 122 may limit absorption, adsorption, and solubility of the bodily fluids in the porous material 122 thereby decreasing the amount of bodily fluids held in the porous material 122. In an embodiment, at least a portion of the porous material 122 is hydrophobic or hydrophilic. In an embodiment, the fluid permeable inner layer is more hydrophobic (e.g., exhibits a larger contact angle with water) than the fluid permeable outer layer. The lower hydrophobicity of the fluid permeable outer layer may help the porous material 122 receive the bodily fluids from the urethral opening while the hydrophobicity of the fluid permeable inner layer limits the bodily fluids that are retained in the porous material 122.

The porous material 122 includes a top end region 140 and a bottom end region 142. The top end region 140 is positioned adjacent to (e.g., covers) the opening 114 or is otherwise positioned closer to the opening 114 defined by the fluid impermeable barrier 106 than the bottom end region 142. The bottom end region 142 may be positioned adjacent to or otherwise positioned closer to the fluid outlet 118 or an inlet of a conduit positioned through the fluid outlet 118. The top end region 140 includes the portions of the porous material 122 within the portions of the chamber 112 defined by the proximal end region 116 of the fluid impermeable barrier 106 and the portions of the porous material 122 extending across the opening 114. The bottom end region 142 includes the portions of the porous material 122 within the portions of the chamber 112 that are defined by the distal end region 120. In an example, the bottom end region 142 is adjacent to and/or proximate to the cap 130 and the edges 113 of the fluid impermeable barrier 106 extending from the cap 130 that are not parallel to a longitudinal axis of the fluid impermeable barrier 106. In an example, the bottom end region 142 may extend about 5 cm or less from the terminal bottom end of the porous material 122, such as about 5 mm or less, about 7.5 mm or less, about 1 cm or less, about 1.25 cm or less, about 1.5 cm or less, about 2 cm or less, about 2.5 cm or less, about 3 cm or less, about 3.5 cm or less, about 4 cm or less, or in ranges of about 5 mm to about 1 cm, about 7.5 mm to about 1.25 cm, about 1 cm to about 1.5 cm, about 1.25 cm to about 2 cm, about 1.5 cm to about 2.5 cm, about 2 cm to about 3 cm, about 2.5 cm to about 3.5 cm, about 3 cm to about 4 cm, or about 3.5 cm to about 5 cm. The porous material may also include an intermediate region 144 extending between the top end region 140 and the bottom end region 142.

The porous material 122 may include at least one top surface 150 and at least one bottom surface 152 opposite the top surface 150. The top surface 150 may extend adjacent to and abut the top panel 108 (when any gaps are removed) and the bottom surface 152 may extend adjacent to and abut the bottom panel 110. The porous material 122 may include one or more edges 154 extending from the top surface 150 to the bottom surface 152 around the periphery of the porous material 122.

As previously discussed, at least a portion of the porous material 122 is attached to the top panel 108 and the bottom panel 110. Attaching the porous material 122 to both the top and bottom panels 108, 110 prevents or at least inhibits gaps forming between the porous material 122 and the top and bottom panels 108, 110 through which the suction may preferably remove air from the gap instead of pulling air and bodily fluids from the porous material 122.

For example, **FIG. 1E** is an enlarged cross-sectional view of the fluid collection assembly 100 taken from circle 1E of **FIG. 1C** before the porous material 122 is attached to the top and bottom panels 108, 110. Since the porous material 122 is not attached to the top and bottom panels 108, 110, the top and bottom panels 108, 110 and the porous material 122 are free to move relative to each other. For instance, **FIG. 1E** illustrates the top panel 108 bowing away from the porous material 122 to form a first gap 146a, the porous material 122 moving away from the bottom panel 108 and the flange 138 to form a second gap 146b, and the porous material 122 moving away from the fluid outlet 118 (e.g., moving away from the inlet of the passageway 136 defined by the cap 130).

**FIG. 1E** also illustrates the dominate fluid flow A (illustrated schematically using arrow A) through the chamber 112 if a suction was applied to the chamber 112 before the porous material 122 is attached to the top and bottom panels 108, 110. As shown, the dominate fluid flow A moves around the porous material 122 such that the suction provided to the chamber 112 preferably removes air from the first and second gaps 146a, 146 instead of removing air and bodily fluids from the porous material 122. The dominate air flow A delays or at least inhibits removal of the bodily fluids from the chamber 112. It is noted that attaching the porous material 122 to only one of the top or bottom panel 108, 110 would still allow the formation of one of the first or second gaps 146a, 146b, respectively such that the suction provided to the chamber 112 still preferably removes air from such gaps instead of air and bodily fluids from the porous material 122. Further, the dominate air flow A may pull the top panel 108 towards the fluid outlet 118 *(i.e.,* into the third gap 146c) which may cause the top panel 108 to at least partially obstruct the fluid outlet 118. It is noted that the flange 138 prevents or at least inhibits the bottom panel 110 from being pulled towards the fluid outlet 118 *(e.g.,* into the third gap 146c). However, without the flange 138, the dominate air flow A may pull the bottom panel 110 towards the fluid outlet 118 such that the bottom panel 110 at least partially obstructs the fluid outlet 118.

**FIG. 1F** is an enlarged cross-sectional view of the fluid collection assembly 100 taken from circle 1E of **FIG. 1C** after the porous material 122 is attached to the top and bottom panels 108, 112. The porous material 122 is attached to the top and bottom panels 108, 110 using one or more porous material seal 148 (shown schematically in **FIGS. 1A** and **1B** using a line and in **FIGS. 1C-1F** using bolded lines). The top and bottom panels 108, 110 and the porous material 122 at least near the porous material seal 148 are not free to move relative to each other since the porous material 122 is attached to the top and bottom panels 108, 110. As such, the porous material seal 148 prevent or at least inhibit the formation of the gaps illustrated in **FIG.** 1E. For example, **FIG. 1F** illustrates the dominate fluid flow B (illustrated schematically using arrow B) through the chamber 112 caused by the suction provided to the chamber 112. As shown, the dominate fluid flow B flows through at least a portion of the porous material 122. The dominate fluid flow B that extends through the porous material 122 pulls air from the porous material 122 (when the pores of the porous material 122 are at least partially occupied by air) which promotes flow of the bodily fluids through the porous material 122 towards the fluid outlet 118. The dominate fluid flow B that extends through the porous material 122 also pulls bodily fluids from the porous material 122 when the pores of the porous material 122 are at least partially occupied by the bodily fluids.

It is noted that gaps may form between the porous material 122 and one or more of the top panel 108 or the bottom panel 108 downstream from the porous material seal 148 *(i.e.,* between the porous material seal 148 and the opening 114). Such gaps may include, for example, the penis receiving area 126. The dominate fluid flow B may predominately flow through the gaps downstream of the porous material seal 148 and may preferentially remove air from the gaps downstream of the porous material seal 148 rather than flow through the portions of the porous material 122 downstream from the porous material seal 148. However, removing the air from the gaps downstream of the porous material seal 148 does not prevent or inhibit removing bodily fluids from the porous material 122 unlike the first, second, and third gaps 146a, 146b, 146c since the dominate fluid flow B still flows through a portion of the porous material 122. For example, the dominate fluid flow B still flows through the porous material 122 around the porous material seal 148 thereby removing any bodily fluids in such portions of the porous material 122. The bodily fluids are attached together via hydrogen bonding since the bodily fluids include water. As such, removing the bodily fluids in the portions of the porous material 122 adjacent to the porous material seal 148 pulls additional bodily fluids into the portions of the porous material 122 adjacent to the porous material seal 148. Additionally, removing air from the gaps downstream of the porous material seal 148 generally decreases the volume of such gaps. The decreased volume of the gaps downstream of the porous material seal 148 prevents or inhibits bodily fluids detrimentally pooling in such gaps and promotes the bodily fluids being beneficially received into the porous material 122.

It is noted that the description of **FIGS. 1E** and **1F** refer to and illustrate the bodily fluids and the dominate fluid flow as flowing towards the fluid outlet 118 since the inlet of the conduit 128 does not extend into the chamber 112. However, it is noted that the bodily fluids and dominate fluid flow would flow towards and into the inlet of the conduit 128 if the conduit 128 was positioned within the chamber 112.

The porous material seal 148 may include a suitable seal that attaches the porous material 122 to the top and bottom panels 108, 110 in a manner than prevents or at least inhibits movement between the top and bottom panels 108, 110 and the porous material 122. The porous material seal 148 may also prevent or at least inhibit air flow therethrough to prevent the porous material seal 148 from forming a passageway through which air may flow without flowing through the porous material 122. The porous material seal 148 may be formed *(i.e.,* the porous material 122 may be attached to the top and bottom panels 108, 110) using any suitable technique. In an example, the porous material seal 148 may include an adhesive (e.g., a silicone adhesive, a hydrogel adhesive, a hotmelt adhesive, or any other suitable adhesive) that attaches the porous material 122 to the top and bottom panels 108, 110. The adhesive should be configured to prevent or limit the adhesive extending into the porous material 122 since the adhesive extending into the porous material 122 may block or otherwise obstruct the pores of the porous material 122 through which bodily fluids may flow. Controlling at least one of the quantity of the adhesive, the contact angle between the adhesive and the porous material 122, or the pressure exerted onto the top and bottom panels 108, 110 and the porous material 122 when attaching these components together may prevent or inhibit the adhesive extending into the porous material 122. In an example, the porous material seal 148 may be formed using a heat weld, US weld, RF weld, or any other suitable weld. The formation of the weld may be controlled to inhibit the distance that the weld extends into the porous material 122 since the weld may block or otherwise obstruct the pores of the porous material 122 through which the bodily fluids may flow. In an example, the porous material seal 148 may be formed from stitching extending through or at least partially through the porous material 122. Unlike the adhesive or welds discussed above, the stitching may minimally obstruct the pores of the porous material 122. It is noted that the porous material seal 148 may be formed from another technique other than an adhesive, welds, or stitches or may be formed using a combination of the above techniques. For example, the porous material seal 148 may be formed from a weld that is reinforced using stitches since the limited distance that the weld may extend into the porous material 122 limits the strength of the weld.

The top surface 150 may be attached to the top panel 108 using a first porous material seal and the bottom surface 152 may be attached to the bottom panel 110 using a second porous material seal. In an embodiment, the first porous material seal and the second porous material seal are the same (e.g., the first and second porous material seal are formed using the same adhesive, the same type of weld, or with stitches extending through the porous material) In an embodiment, the first porous material seal and the second porous material seal are the different. The first porous material seal and the second porous material seal may be different for a variety of reasons. In an example, one of the first porous material seal or the second porous material seal may be formed while manufacturing the fluid collection assembly 100 and the other of the first porous material seal or the second porous material seal may be formed during a retrofit of the fluid collection assembly 100.

As previously discussed, the top panel 108 and the bottom panel 110 are attached together using the panel seal 113 when the top panel 108 and the bottom panel 110 are distinct. In an embodiment, the panel seal 113 and the porous material seal 148 may be distinct from each other. The panel seal 113 and the porous material seal 148 may be distinct from each other when the panel seal 113 and the porous material seal 148 are at least one of visually distinguishable from each other or formed separately from each other. In an example, the panel seal 113 and the porous material seal 148 are distinct from each other when the panel seal 113 and the porous material seal 148 are spaced from each other. In an example, the panel seal 113 and the porous material seal 148 are distinct from each other because the attachment used to form the panel seal 113 is different than the attachment used to form the porous material seal 148. For instance, in such an example, the panel seal 113 may be formed from a first weld (e.g., one of heat welding, US welding, RF welding, etc.) and the porous material seal 148 is at least one of formed from a second weld that is different from the first weld, formed using an adhesive, or formed using stitching. In an example, the panel seal 113 is distinct from the porous material seal 124 when the panel seal 113 is formed either before or after the porous material seal 148.

The panel seal 113 and the porous material seal 148 may be distinct from each other for a variety of reasons. In an example, the fluid impermeable barrier 106 and the porous material 122 may be formed from different materials which require different attachment techniques. In an example, the porous material 122 is formed from a porous material that needs at least some of the pores thereof to remain open to function while the fluid impermeable barrier 106 does not. As such, in such an example, the panel seal 113 may be formed using a technique that would otherwise obstruct the pores of the porous material 122.

In an embodiment, at least one of substantially all of the top surface 150 is attached to the top panel 108 or substantially all of the bottom surface 152 is attached to the bottom panel 110 using the porous material seal 148. In such an embodiment, it is difficult for gaps to form between the surface of the porous material 122 and the top panel 108 and/or the bottom panel 110. Further attaching substantially all of the top surface 150 and/or the bottom surface 152 to their respective panel strengthens the attachment therebetween.

In an embodiment, as shown, only a portion of at least one of the top surface 150 is attached to the top panel 108 or the bottom surface 152 is attached to the bottom panel 110 via the porous material seal 148. For example, only the portion of the top surface 150 at or near the edges 154 of the porous material 122 are attached to the top panel 108 and/or only the portion of the bottom surface 152 at or near the edges 154 of the porous material 122 are attached to the bottom panel 110. In such an example, the porous material seal 148 is still able to ensure that the dominate fluid flow flows through at least a portion of the porous material 122 while also allowing the fluid collection assembly 100 to include certain gaps, such as the penis receiving area 126. Further only attaching portions of the top surface 150 to the top panel 108 and/or the bottom surface 152 to the bottom panel 110 decreases the likelihood that the porous material seal 148 at least partially obstruct the pores of the porous material 122. Additionally, it may be easier to attach only portions of the top surface 150 to the top panel 108 and/or the bottom surface 152 to the bottom panel 110 than if substantially all of the portions of the top surface 150 is attached to the top panel 108 and/or substantially all of the bottom surface 152 is attached to the bottom panel 110, respectively.

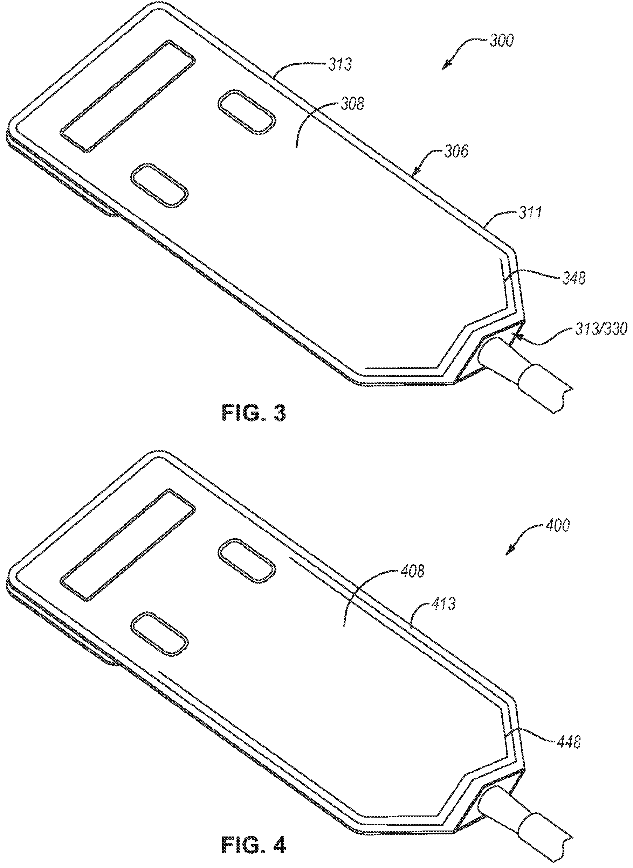

In an embodiment, as shown, the porous material seal 148 may extend along at least substantially an entire periphery of the porous material 122 when only the portions of at least one of the top surface 150 is attached to the top panel 108 or the bottom surface 152 is attached to the bottom panel 110 at or near the edges 154 of the porous material 122. In such an embodiment, the fluid flow in the chamber 112 caused by the suction must flow through the porous material 122 since there is no pathway for the fluid flow to circumvent the porous material seal 148. Such fluid flow maximizes the vacuum-assisting wicking of bodily fluids through the porous material 122. That said, as will be discussed in more detail with regards to **FIGS.** 3 and **4****,** the porous material seal 148 does not need to extend along the entire periphery of the porous material 122.

The porous material seal 148 may be formed at any suitable time. In an example, the porous material seal 148 is at least partially formed (*i.e.,* the porous material 122 is attached to one or both of the top panel 108 or the bottom panel 110) before the panel seal 113 is formed *(i.e.,* before the top and bottom panels 108, 110 are attached together). Such an example may facilitate formation of the porous material seal 148 since the porous material 122 may be more easily accessed before forming the panel seal 113. In an example, the porous material seal 148 may be at least partially formed simultaneously with the panel seal 113. In such an example, forming the porous material seal 148 and the panel seal 113 substantially simultaneously may be advantageous when the porous material seal 148 and the panel seal 113 have the same type of attachment. In an example, at least a portion of the porous material seal 148 may be formed after forming the panel seal 113. In such an example, the porous material seal 148 may be formed during a retrofitting of a conventional fluid collection assembly. Retrofitting conventional fluid collection assemblies to include the porous material seal 148 may allow for in-the-field customizations based on the needs and/or preferences of the patient or caregiver and allows for simpler inventory management since fewer types of fluid collection assemblies.

In an embodiment, the porous material 122 may abut the inlet of the fluid outlet 118. The porous material seal 148 may maintain the porous material 122 against the inlet of the fluid outlet 118 when at least one of substantially all of the top surface 150 is attached to the top panel 108, substantially all of the bottom surface 152 is attached to the bottom panel 110, the top surface 150 is attached to the top panel 108 at or near the edges 154 of the porous material 122, or the bottom surface 152 is attached to the bottom panel 110 at or near the edges 154 of the porous material 122. Maintaining the porous material 122 against the inlet of the fluid outlet 118 may prevent the formation of the third gap 146c illustrated in FIG. 1E. In other words, maintaining the porous material 122 against the inlet of the fluid outlet 118 may prevent the suction pulling the top panel 108 and/or the bottom panel 110 towards to the inlet of the fluid outlet 118 such that the top panel 108 and/or the bottom panel 110 at least partially obstructs the inlet of the fluid outlet 118. In an embodiment, the porous material 122 does not abut the inlet of the fluid outlet 118. In such an embodiment, the fluid impermeable barrier 106 may exhibit sufficient rigidity that the suction is unlikely to pull top panel 108 and/or the bottom panel 110 towards to the inlet of the fluid outlet 118. The gap between the porous material 122 and the inlet of the fluid outlet 118 effectively forms an extension of the conduit 128 extending between the inlet of the fluid outlet 118 to the porous material 122. In an embodiment, the porous material 122 does not extend into or through the fluid outlet 118.

Generally, the sheath 102 is substantially flat when the penis is not in the penis receiving area 126 and the sheath 102 is resting on a flat surface. The sheath 102 is substantially flat because the fluid impermeable barrier 106 is formed from the top panel 108 and the bottom panel 110 instead of a generally tubular fluid impermeable barrier. Further, as previously discussed, the porous material 122 may be a sheet, which also causes the sheath 102 to be substantially flat. The sheath 102 may also be substantially flat because the fluid collection assembly 100 may not include relatively rigid rings or caps that exhibit a rigidity that is greater than the portions of the fluid impermeable barrier 106 thereabout since such rings and caps may inhibit the sheath 102 being substantially flat. It is noted that the sheath 102 is described as being substantially flat because at least one of the porous material 122 may cause a slight bulge to form in the sheath 102 depending on the thickness of the porous material 122, the fluid outlet 118 and/or conduit 128 may cause a bulge thereabout, or the base 104 may pull on portions of the sheath 102 thereabout. It is also noted that the sheath 102 may also be compliant and, as such, the sheath 102 may not be substantially flat during use since, during use, the sheath 102 may rest on a non-flat surface (e.g., may rest on the testicles, the perineum, and/or between the thighs) and the sheath 102 may conform to the surface of these shapes.

The ability of the sheath 102 to be substantially flat when the penis is not in the penis receiving area 126 and the sheath 102 is resting on a flat surface allows the fluid collection assembly 100 to be used with a buried and a non-buried penis. For example, when the fluid collection assembly 100 is being used with a buried penis, the penis does not extend into the penis receiving area 126 which causes the sheath 102 to lie relatively flat across the aperture 156 of the base 104. When the sheath 102 lies relatively flat across the aperture 156, the porous material 122 extends across the opening 114 and the aperture 156 and is in close proximity to the buried penis. As such, the porous material 122 prevents or inhibits pooling of bodily fluids discharged from the buried penis against the skin of the individual since the porous material 122 will receive and remove at least a significant portion of the bodily fluids that would otherwise pool against the skin of the individual. Thus, the skin of the individual remains dry thereby improving comfort of using the fluid collection assembly 100 and preventing skin degradation. However, unlike other conventional fluid collection assemblies that are configured to be used with buried penises, the fluid collection assembly 100 may still be used with a non-buried penis since the non-buried penis can still be received into the penis receiving area 126, even when the penis is fully erect. Additionally, the ability of the sheath 102 to be substantially flat allows the fluid collection assembly 100 to be used more discretely than if the sheath 102 was not substantially flat thereby avoiding possibly embarrassing scenarios.

When the sheath 102 is substantially flat, the porous material 122 occupies substantially all of the chamber 112 and the penis receiving area 126 is collapsed (shown as being non-collapsed in **FIGS. 1C** and **1D** for illustrative purposes to show the penis receiving area 126). In other words, the sheath 102 may not define a region that is constantly unoccupied by the porous material 122. When the porous material 122 occupies substantially all of the chamber 112, the bodily fluids discharged into the chamber 112 are unlikely to pool for significant periods of time since pooling of the bodily fluids may cause sanitation issues, cause an odor, and/or may cause the skin of the individual to remain in contact with the bodily fluids which may cause discomfort and skin degradation.

As previously discussed, the top panel 108, the bottom panel 110, and the porous material 122 may be selected to be relatively flexible. The top panel 108, the bottom panel 110, and the porous material 122 are relatively flexible when the top panel 108, the bottom panel 110, and the porous material 122, respectively, are unable to maintain their shape when unsupported. The flexibility of the top panel 108, the bottom panel 110, and the porous material 122 may allow the sheath 102 to be substantially flat, as discussed above. The flexibility of the top panel 108, the bottom panel 110, and the porous material 122 may also allow the sheath 102 to conform to the shape of the penis even when the size and shape of the penis changes (e.g., becomes erect) and to minimize any unoccupied spaces in the chamber 112 in which bodily fluids may pool.

As previously discussed, the fluid collection assembly 100 includes a base 104 that is configured to be attached to the sheath 102. For example, the base 104 is configured to be permanently attached to the sheath 102. The base 104 is configured to be permanently attached to the sheath 102 when, for example, when the fluid collection assembly 100 is provided with the base 104 permanently attached to the sheath 102 or the base 104 is provided without being permanently attached to the sheath 102 but is configured to be permanently attached to the sheath 102 at some point in the future. Permanently attached means that the sheath 102 cannot be detached from the base 104 without damaging at least one of the sheath 102 or the base 104, using a blade to separate the sheath 102 from the base 104, and/or using chemicals to dissolve the adhesive that attaches the sheath 102 from the base 104. The base 104 may be permanently attached to the sheath 102 using an adhesive, sewing, heat sealing, RF welding, or US welding. In an embodiment, the base 104 is configured to be reversibly attached to the sheath 102. In an embodiment, the base 104 is integrally formed with the sheath 102.

The base 104 includes an aperture 156. The base 104 is permanently attached to the distal end region 120 of the sheath 102 such that the aperture 156 is aligned with the opening 114.

The base 104 is sized, shaped, and made of a material to be coupled to the skin that surrounds the penis (e.g., mons pubis, thighs, testicles, and/or perineum) and have the penis disposed therethrough. For example, the base 104 may define an aperture 156 configured to have the penis positioned therethrough. In an example, the base 104 may exhibit the general shape or contours of the skin surface that the base 104 is configured to be coupled with. The base 104 may be flexible, thereby allowing the base 104 to conform to any shape of the skin surface and mitigate the base 104 pulling the on skin surface. The base 104 may extend laterally past the sheath 102 thereby increasing the surface area of the skin of the individual to which the fluid collection assembly 100 may be attached compared to a substantially similar fluid collection assembly 100 that did not include a base.

As previously discussed, the fluid collection assembly 100 includes the conduit 128. The inlet of the conduit 128 may be located near the distal end region 120 of the sheath 102 which is expected to be the gravimetrically low point of the chamber 112 when worn by an individual. The inlet of the conduit 128 may be located near the distal end region 120 of the sheath 102 when the cap 130 is located at or near the distal end region 120. Locating the inlet at or near the distal end region 120 of the sheath 102 enables the conduit 128 to receive more of the bodily fluids than if the inlet of the conduit 128 was located elsewhere and reduce the likelihood of pooling (e.g., polling of the bodily fluids may cause microbe growth and foul odors).

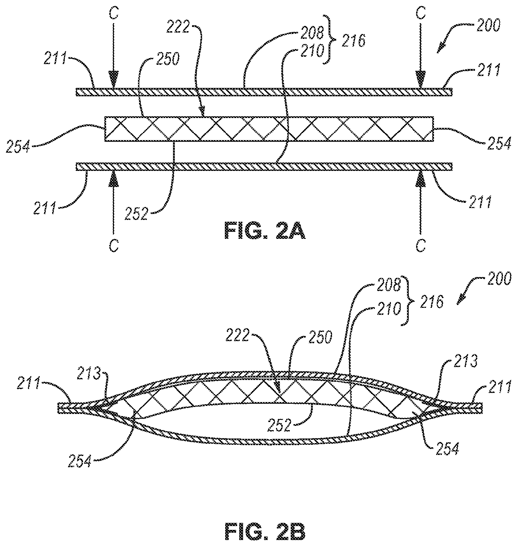

As previously discussed, the panel seal 113 and the porous material seal 148 of the fluid collection assembly 100 illustrated in **FIGS. 1A-1F** are distinct from each other. However, the panel seals and the porous material seal of the fluid collection assemblies disclosed herein may be the same. That is, the panel seals and the porous material seal disclosed herein may not be distinct from each other. **FIG. 2A** is a cross-sectional view of a method of forming a fluid collection assembly 200, according to an embodiment. **FIG. 2B** is a cross-sectional view of the fluid collection assembly 200 after being formed. Except as otherwise disclosed herein, the fluid collection assembly 200 is the same as or substantially similar to any of the fluid collection assemblies disclosed herein. For example, the fluid collection assembly 200 includes a fluid impermeable barrier 206 including a top panel 208 and a bottom panel 210. The fluid collection assembly 200 also includes at least one porous material 222.

The fluid collection assembly 200 is formed by providing the top panel 208, the bottom panel 210, and the porous material 222. The top panel 208 and bottom panel 210 include edges 211. The porous material 222 includes a top surface 250, a bottom surface 252, and edges 254. The porous material 222 is positioned between the top panel 208 and the bottom panel 210 such that the top surface 250 of the porous material 222 is adjacent to the top panel 208 and the bottom surface 232 is adjacent to the bottom panel 210.

The edges 211 of the top and bottom panels 208, 210 may be brought together by applying a force C (shown schematically with arrows). The edges 254 of the porous material 222 may be pinched together and compressed when the edges 211 of the top and bottom panels 208, 210 are brought together. It is noted that the porous material 222 may exhibit a width that is less than the width of the top and bottom panels 208, 210 such that applying the force C to the top and bottom panels 208, 210 causes a portion of the top and bottom panels 208, 210 to directly contact each other and another portion of the top and bottom panels 208, 210 to directly contact the porous material 222. The edges 211 of the top and bottom panels 208, 210 and the areas thereabout may be attached together and to the porous material 222 to form a panel and porous material seal 213. The panel and porous material seal 213 may be formed using any of the techniques disclosed herein. For example, the panel and porous material seal 213 may be formed using welds, adhesives, stitches, or any other suitable attachment technique. It is noted that the panel and porous material seal 213 may extend along substantially an entirety of a periphery of the porous material 222 or, as will be discussed in more detail below, a portion of a periphery of the porous material 222.

The panel and porous material seal 213 may quicken and simplify manufacturing of the fluid collection assembly 200 compared to the fluid collection assembly 100 shown in **FIGS. 1A-1F** since the fluid collection assembly 200 only needs to form one seal instead of two distinct seals. However, the compressed porous material 222 provides a stress to the panel and porous material seal 213 that is continuously attempted to break the panel and porous material seal 213 thereby increasing the likelihood that gaps forms between the top and bottom panels 208, 210 through which bodily fluids may leak. It is further noted that, depending on the technique used to form the panel and porous material seal 213, the panel and porous material seal 213 may at least partially obstruct more pores of the porous material 222 thereby decreasing the quantity of bodily fluids that may flow through and be temporarily stored in the porous material 222 compared to the porous material 122 of **FIGS. 1A-1F** assuming the starting volume of the porous materials 122 and the porous material 222 are the same.

Referring back to **FIGS. 1A** and **1B****,** the porous material seal 148 extends along substantially all of an outer periphery of the porous material 122. However, the porous material seal disclosed herein, including the panel and porous material seal, may extend along only a portion of an outer periphery of the porous material. **FIGS. 3** and **4** are top isometric views of different fluid collection assemblies having a porous material seal extending along only a portion of an outer periphery of the porous material, according to different embodiments. Except as otherwise disclosed herein, the fluid collection assemblies illustrated in **FIGS.** 3 and **4** are the same as or substantially similar to any of the fluid collection assemblies disclosed herein.

Referring to **FIG.** 3, only the portions of the bottom end region of the porous material (not shown, obscured by the top panel 308) are attached to the top panel 308 and the bottom panel (not shown, obscured). The porous material seal 348 that attaches the bottom end region of the porous material to the top panel 308 and the bottom panel is shown schematically with a line in **FIG.** 3. The portions of the bottom end region of the porous material that are attached to the top panel 308 and the bottom panel include the portions of the porous material adjacent to the fluid outlet 318 (e.g., the cap 330), adjacent to the edges of the fluid impermeable barrier 306 extending from the fluid outlet 318 *(e.g.,* the edges 311 of the fluid impermeable barrier 306 that are not parallel to the longitudinal axis of the fluid impermeable barrier 306), or any other portion of the bottom end region. In an example, only the portions of the bottom end region of the porous material at or near the edges of the porous material are attached to the top panel 308 and the bottom panel. The bottom end region of the porous material may be attached to the top panel 308 and the bottom panel using any of the techniques disclosed herein. For example, the portion of the bottom end region of the porous material may be attached to the top panel 308 and the bottom panel using a porous material seal 348 that is distinct from panel seal 313.

Attaching only the portions of the bottom end region of the porous material to the top panel 308 and the bottom panel may facilitate manufacturing of the fluid collection assembly 300 by decreasing the size of the porous material seal 348 compared to the porous material seal 148 shown in **FIGS. 1A-1F****.** Also, only attaching the portions of the bottom end region to the top panel 308 and the bottom panel decreases the amount of pores that are inadvertently obstructed by the adhesive, weld, etc. used to form the porous material seal 348 than if more of the porous material was attached to the top panel 308 and the bottom panel.

In an embodiment, the panel seal 313 and the porous material seal 348 are distinct from each other. In such an embodiment, at least some of the fluid flow caused by the suction provided to the chamber may flow between the panel seal 313 and the porous material seal 348. The fluid flow between the panel seal 313 and the porous material seal 348 may decrease the vacuum-assisted wicking of the bodily fluids in the porous material compared to the vacuum-assisted wicking of the bodily fluids in the porous material 122 shown in **FIGS. 1C-1F****.** However, the decrease in the vacuum-assisted wicking of the bodily fluids in the porous material may be negligible or otherwise minor due to the torturous path that fluid must flow to get around the porous material seal 348.

Referring to **FIG. 4****,** the bottom end region and at least a portion of the intermediate portion of the porous material (not shown, obscured by the top panel 408) is attached to the top panel 408 and the bottom panel (not shown, obscured) via the porous material seal 448. In an embodiment, the top end region of the porous material is not attached to the top panel 408 and the bottom panel. In an embodiment, a portion of the top end region of the porous material may be attached to the top panel 408 and the bottom panel.

Attaching the portions of the bottom end region and at least a portion of the intermediate portion of the porous material to the top panel 408 and the bottom panel may facilitate manufacturing of the fluid collection assembly 400 by decreasing the size of the porous material seal 448 compared to the porous material seal 148 shown in **FIGS. 1C****,** **1D,** and **1F****.** Also, attaching the portions of the bottom end region and the intermediate portion to the top panel 408 and the bottom panel decreases the amount of pores that are inadvertently blocked by the adhesive, weld, etc. used to form the porous material seal 448 than if more of the porous material was attached to the top panel 308 and the bottom panel.

In an example, the portions of the bottom end region of the porous material and the intermediate portion at or near the edges of the porous material are attached to the top panel 408 and the bottom panel. The portion of the bottom end region of the porous material may be attached to the top panel 408 and the bottom panel using any of the techniques disclosed herein. For example, the portion of the bottom end region of the porous material may be attached to the top panel 408 and the bottom panel using a porous material seal 448 that is distinct from panel seal 413.

In an embodiment, the panel seal 313 and the porous material seal 348 are distinct from each other. In such an embodiment, at least some of the fluid flow caused by the suction provided to the chamber may flow between the panel seal 313 and the porous material seal 348. The fluid flow between the panel seal 313 and the porous material seal 348 may decrease the vacuum-assisted wicking of the bodily fluids in the porous material compared to the vacuum-assisted wicking of the bodily fluids in the porous material 122 shown in **FIGS. 1C-1F****.** However, the decrease in the vacuum-assisted wicking of the bodily fluids in the porous material of the fluid collection assembly 400 may be less than the decrease in the vacuum assisted wicking of the bodily fluids in the porous material of the fluid collection assembly 300 shown in **FIG.** 3.

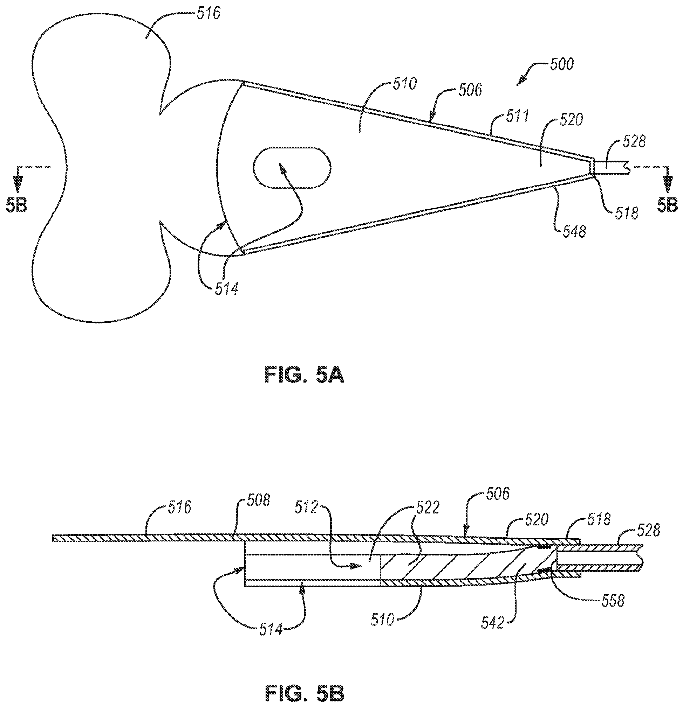

**FIG. 5A** is a bottom plan view of a fluid collection assembly 500, according to an embodiment. **FIG. 5B** is a cross-sectional view of the fluid collection assembly 500 taken along plane 5B-5B shown in **FIG. 5A****.** The fluid collection assembly 500 is an example of a male fluid collection assembly though, it is noted, the fluid collection assembly 500 may be used with females. Except as otherwise disclosed herein, the fluid collection assembly 500 is the same as or substantially similar to any of the fluid collection assemblies disclosed herein. Further, any of the features of the fluid collection assembly 500 may be used with any of the fluid collection assemblies disclosed herein.

The fluid collection assembly 500 includes a fluid impermeable barrier 506. The fluid impermeable barrier 506 may be the same as or substantially similar to any of the fluid impermeable barriers disclosed herein. The fluid impermeable barrier 506 includes a top panel 508 and a bottom panel 510. The fluid impermeable barrier 506 at least partially defines an opening 514 and a chamber 512 within the fluid collection assembly 500 in fluid communication with the opening 514. In some embodiments, a bottom panel of the fluid impermeable barrier 506 may define the opening 514. The fluid impermeable barrier 506 includes a proximal end region 516 (e.g., one or more wings configured to be attached to the patient) and a distal end region 520. The opening 514 may be positioned proximate or closer to the proximal end region 516 than the distal end region 520 of the fluid impermeable barrier 506. The fluid impermeable barrier 506 also defines fluid outlet 518 may be positioned proximate or closer to the distal end region 520 than the proximal end region 516. In some embodiments, the fluid impermeable barrier 506 narrows between the proximal end region 516 and the distal end region 520. For example, the fluid impermeable barrier 506 (and the chamber 512) may include a substantially triangular front profile, with the distal end region 520 being at the narrow end or tip of the triangular profile. The fluid impermeable barrier 506 may include a shape substantially complementary to the chamber 512, such as a substantially triangular front profile. The fluid impermeable barrier 506 may include a substantially flexible fluid impermeable material, such as a fluid impermeable polymer (e.g., silicone, polypropylene, polyethylene, polyethylene terephthalate, a polycarbonate, etc.), polyurethane films, thermoplastic elastomer, oil, another suitable material, or combinations thereof. In some embodiments, the fluid impermeable barrier includes a paper-like or bag-like fluid impermeable material and/or a fluid impermeable fabric.

The fluid collection assembly 500 also includes a porous material 522 positioned within the chamber 512 and extending at least partially between the distal end region 520 and the proximal end region 516. The porous material 522 may be shaped generally complementary to the shape of the chamber of the fluid impermeable barrier 506. In some embodiments, the porous material 522 is spaced from the edges 511 of the fluid impermeable barrier 506 that extend at least partially between the distal end region 520 and the proximal end region 516. In some embodiments, the porous material 522 is positioned to abut the edges 511 of the fluid impermeable barrier 506 such that fluid impermeable barrier 506 retains fluid in the porous material 522 from the opening 514 to the fluid outlet 518.

The fluid collection assembly 500 includes at least one porous material 522. The porous material 522 may be the same as or substantially similar to any of the porous materials disclosed herein. The porous material 522 can remove fluid from the area around the penis, thereby leaving the area and urethra dry. The porous material 522 can enable the fluid to flow generally towards the inlet 558 of the conduit 528. The porous material 522 may be attached to the top panel 508 and the bottom panel 510 of the fluid impermeable barrier 506 using any of the techniques disclosed herein. For example, the porous material 522 may be attached to the top panel 508 and the bottom panel 510 using a porous material seal 548. In some embodiments, the bottom end region 542 of the porous material 522 is shaped substantially complementary to the distal end region 520.

The fluid collection assembly 500 also includes a conduit 528 extending into the chamber 512 and having an end positioned proximate to the distal end region 520 of the fluid impermeable barrier 506. As shown in **FIG. 2B****,** the conduit 528 includes an inlet 558 proximate to the end 218 of the conduit 528. Returning to **FIG. 2A****,** bodily fluids received in the porous material 522 may flow to and pool in the distal end region 520 for removal when a vacuum is applied on the conduit 528. The conduit 528 may extend through the fluid outlet 518 of the fluid impermeable barrier 506 and into the chamber 512. In some embodiments, a first portion of the conduit 528 including the inlet 558 may be fixedly connected to the fluid impermeable barrier 506 proximate to the fluid outlet 518 and a second portion of the conduit 528 may removably secured or connected to the fluid collection device 500 proximate to the fluid outlet 518. The fluid outlet 518 may be positioned proximate to the distal end region 520 of fluid impermeable barrier 506. In some embodiments, the inlet 558 of the conduit 528 is positioned between the distal end region 520 and the fluid outlet 518. In some embodiments, the fluid outlet 518 is absent, and the conduit 528 may exit the chamber 512 through the opening 514. U.S. Provisional Patent 63/067,542 describes embodiments of male external catheter fluid collection assemblies..

Other examples of fluid collection assemblies that may include the porous materials thereof attached to the fluid impermeable barrier are disclosed in U.S. Patent Application No. 18/299,788 filed on April 13, 2023, U.S. Patent No. 11,376,152 filed on November 3, 2020, U.S. Patent Application No. 17/996,155 filed on October 13, 2022, U.S. Patent No. 17/996,064 filed on October 12, 2022, U.S. Patent Application No. 17/664,487 filed on May 23, 2022, PCT International Application No. PCT/US2022/022111 filed on March 28, 2022, U.S. Patent No. 10,376,407 issued on August 13, 2019, U.S. Patent Application No. 16/478,180 filed on January 30, 2018, U.S. Patent No. 10,376,406 issued on August 13, 2019, U.S. Patent Application No. 16/433,773 filed on June 6, 2019, U.S. Patent Application No. 17/614,173 filed on November 24, 2021, U.S. Patent Application No. 18/003,029 filed on December 22, 2022, U.S. Patent Application No. 18/042,842 filed on February 24, 2023, and U.S. Patent Application No. 17/446,654 filed on September 1, 2021.

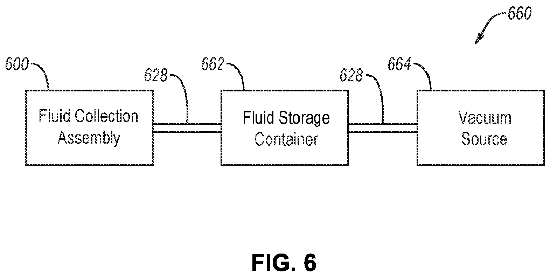

**FIG. 6** is a block diagram of a fluid collection system 660 for fluid collection, according to an embodiment. The fluid collection system 660 includes a fluid collection assembly 600, a fluid storage container 662, and a vacuum source 664. The fluid collection assembly 600 may be the same or substantially similar to any of the fluid collection assemblies disclosed herein. The fluid collection assembly 600, the fluid storage container 662, and the vacuum source 664 may be fluidly coupled to each other via one or more conduits 628. For example, fluid collection assembly 600 may be operably coupled to one or more of the fluid storage container 662 or the vacuum source 664 via the conduit 628. The bodily fluids collected in the fluid collection assembly 600 may be removed from the fluid collection assembly 600 via the conduit 628 which protrudes into the fluid collection assembly 600. For example, an inlet of the conduit 628 may extend into the fluid collection assembly 600, such as to a reservoir therein. The outlet of the conduit 628 may extend into the fluid collection assembly 600 or the vacuum source 664. Suction force may be introduced into the chamber of the fluid collection assembly 600 via the inlet of the conduit 628 responsive to suction (e.g., vacuum) force applied at the outlet of the conduit 628.

The suction force may be applied to the outlet of the conduit 628 by the vacuum source 664 either directly or indirectly. The suction force may be applied indirectly via the fluid storage container 662. For example, the outlet of the conduit 628 may be disposed within the fluid storage container 662 and an additional conduit 628 may extend from the fluid storage container 662 to the vacuum source 664. Accordingly, the vacuum source 664 may apply suction to the fluid collection assembly 600 via the fluid storage container 662. The suction force may be applied directly via the vacuum source 664. For example, the outlet of the conduit 628 may be disposed within the vacuum source 664. An additional conduit 628 may extend from the vacuum source 664 to a point outside of the fluid collection assembly 600, such as to the fluid storage container 662. In such examples, the vacuum source 664 may be disposed between the fluid collection assembly 600 and the fluid storage container 662.

The fluid storage container 662 is sized and shaped to retain bodily fluids therein. The fluid storage container 662 may include a bag (e.g., drainage bag), a bottle or cup (e.g., collection jar), or any other enclosed container for storing bodily fluids such as urine. In some examples, the conduit 628 may extend from the fluid collection assembly 600 and attach to the fluid storage container 662 at a first point therein. An additional conduit 628 may attach to the fluid storage container 662 at a second point thereon and may extend and attach to the vacuum source 664. Accordingly, a vacuum (e.g., suction) may be drawn through fluid collection assembly 600 via the fluid storage container 662. Bodily fluids, such as urine, may be drained from the fluid collection assembly 600 using the vacuum source 664.

The vacuum source 664 may include one or more of a manual vacuum pump, and electric vacuum pump, a diaphragm pump, a centrifugal pump, a displacement pump, a magnetically driven pump, a peristaltic pump, or any pump configured to produce a vacuum. The vacuum source 664 may provide a vacuum or suction to remove bodily fluids from the fluid collection assembly 600. In some examples, the vacuum source 664 may be powered by one or more of a power cord (e.g., connected to a power socket), one or more batteries, or even manual power (e.g., a hand operated vacuum pump). In some examples, the vacuum source 664 may be sized and shaped to fit outside of, on, or within the fluid collection assembly 600. For example, the vacuum source 664 may include one or more miniaturized pumps or one or more micro pumps. The vacuum sources 664 disclosed herein may include one or more of a switch, a button, a plug, a remote, or any other device suitable to activate the vacuum source 664.

While various aspects and embodiments have been disclosed herein, other aspects and embodiments are contemplated. The various aspects and embodiments disclosed herein are for purposes of illustration and are not intended to be limiting, without departing from the scope of the appended set of claims

Terms of degree (e.g., "about," "substantially," "generally," etc.) indicate structurally or functionally insignificant variations. In an example, when the term of degree is included with a term indicating quantity, the term of degree is interpreted to mean ± 10%, ±5%, or +2% of the term indicating quantity. In an example, when the term of degree is used to modify a shape, the term of degree indicates that the shape being modified by the term of degree has the appearance of the disclosed shape. For instance, the term of degree may be used to indicate that the shape may have rounded corners instead of sharp corners, curved edges instead of straight edges, one or more protrusions extending therefrom, is oblong, is the same as the disclosed shape, etc.

## Claims

1. A fluid collection assembly (100), comprising:
a fluid impermeable barrier (106) defining at least a chamber (112), at least one opening, and a fluid outlet (118), the fluid impermeable barrier (106) including a top panel (108) and a bottom panel (110) opposite the top panel (108);
at least one porous material (122) disposed in the chamber (112), the at least one porous material (122) including a top end region (140) and a bottom end region (142);
**characterised in that** edges of at least the bottom end region (142) of the at least one porous material (122) are fixedly attached to the top panel (108) and the bottom panel (110) with a porous material seal (148).

2. The fluid collection assembly according to claim 1, wherein the top panel (108) is distinct from the bottom panel (110) , and wherein the edges of the top panel (108) are attached to the corresponding edges of the bottom panel (110) with a panel seal (113), wherein the porous material seal (148) and the panel seal (113) are the same.

3. The fluid collection assembly according to claim 1, wherein the top panel (108) is distinct from the bottom panel (110), and wherein the edges of the top panel (108) are attached to the corresponding edges of the bottom panel (110) with a panel seal (113), wherein the porous material seal (148) and the panel seal (113) are distinct and separate from each other.

4. The fluid collection assembly according to claim 3, wherein the porous material seal (148) and the panel seal (113) include different types of attachments.

5. The fluid collection assembly according to any of preceding claims, wherein only the bottom end region of the at least one porous material (122) is attached to the top panel (108) and the bottom panel (110).

6. The fluid collection assembly according to any of preceding claims, wherein the top end region (140) of the at least one porous material (122) is not attached to the top panel (108) and the bottom panel (110).

7. The fluid collection assembly according to any of preceding claims, wherein substantially all of the edges of the at least one porous material (122) are fixedly attached to the top panel (108) and the bottom panel (110).

8. The fluid collection assembly according to any of preceding claims, wherein the porous material seal (148) is configured to maintain the contact between the at least one porous material (122) and the fluid outlet (118) or an inlet of the conduit.

9. The fluid collection assembly according to any of preceding claims, wherein the porous material seal (148) includes an adhesive attaching the at least one porous material (122) to at least one of the top panel (108) or the bottom panel (110).

10. The fluid collection assembly according to any of preceding claims, wherein the porous material seal includes a weld attaching the at least one porous material (122) to at least one of the top panel (108) or the bottom panel (110).

11. The fluid collection assembly according to any of preceding claims, wherein the porous material seal (148) includes a stitch attaching the at least one porous material (122) to at least one of the top panel (108) or the bottom panel (110).

12. The fluid collection assembly of any one of according to any of preceding claims, wherein at least the bottom end region of the at least one porous material (122) is attached to the top panel (108) using a first porous material seal and the bottom panel (110) using a second porous material seal, and wherein the first porous material seal is different from the second porous material seal.

13. A fluid collection system (660), comprising:
the fluid collection assembly of any one of claims 1-12;
a fluid storage container (662); and
a vacuum source (664);
wherein the chamber of the fluid collection assembly, the fluid storage container (662), and the vacuum source (664) are in fluid communication with each other so that, when one or more bodily fluids are present in the chamber, a suction provided from the vacuum source (664) to the chamber of the fluid collection assembly removes the one or more bodily fluids from the chamber and deposits the bodily fluids in the fluid storage container (662).

14. A method of forming a fluid collection assembly, according to claim any of claims 1-12, the method comprising:
providing at least one porous material (122) disposed in a chamber (112) defined by a fluid impermeable barrier (106), the fluid impermeable barrier (106) defining at least the chamber (112), at least one opening, and a fluid outlet (118), the fluid impermeable barrier (106) including a top panel (108) and a bottom panel (110) opposite the top panel (108), the at least one porous material (122) including a top end region and a bottom end region; and
fixedly attaching the at least one porous material to at least one of the top panel (108) or the bottom panel (110) with a porous material seal (148).

15. The method of claim 14, wherein providing at least one porous material (122) disposed in the chamber includes providing the at least one porous material (122) positioned between the top panel (108) and the bottom panel (110), wherein the top panel (108) and the bottom panel (110) are not attached together; and
wherein fixedly attaching the at least one porous material (122) to at least one of the top panel (108) or the bottom panel (110) with the porous material seal (148) includes fixedly attaching edges of the top panel (108) to the bottom panel (110) and attaching the at least one porous material (122) to the top panel (108) and the bottom panel (110) simultaneously.

16. The method of claim 14, wherein:
providing at least one porous material (122) disposed in the chamber (112) includes providing the at least one porous material (122) attached to one of the top panel (108) or the bottom panel (110); and
fixedly attaching the at least one porous material (122) to at least one of the top panel (108) or the bottom panel (110) includes fixedly attaching the at least one porous material (122) to the other of the top panel (108) or the bottom panel (110).

## Patentansprüche

1. Flüssigkeitssammelbaugruppe (100), umfassend:
eine flüssigkeitsundurchlässige Barriere (106), die mindestens eine Kammer (112), mindestens eine Öffnung und einen Flüssigkeitsauslass (118) definiert, wobei die flüssigkeitsundurchlässige Barriere (106) eine obere Platte (108) und eine der oberen Platte (108) gegenüberliegende untere Platte (110) beinhaltet;
mindestens ein poröses Material (122), das in der Kammer (112) angeordnet ist, wobei das mindestens eine poröse Material (122) einen oberen Endbereich (140) und einen unteren Endbereich (142) beinhaltet;
**dadurch gekennzeichnet, dass**
Ränder zumindest des unteren Endbereichs (142) des mindestens einen porösen Materials (122) mit einer Dichtung aus porösem Material (148) fest an der oberen Platte (108) und der unteren Platte (110) befestigt sind.

2. Flüssigkeitssammelbaugruppe nach Anspruch 1, wobei die obere Platte (108) von der unteren Platte (110) unterschiedlich ist und wobei die Ränder der oberen Platte (108) an den entsprechenden Rändern der unteren Platte (110) mit einer Plattendichtung (113) befestigt sind, wobei die Dichtung aus porösem Material (148) und die Plattendichtung (113) identisch sind.

3. Flüssigkeitssammelbaugruppe nach Anspruch 1, wobei die obere Platte (108) von der unteren Platte (110) unterschiedlich ist und wobei die Ränder der oberen Platte (108) an den entsprechenden Rändern der unteren Platte (110) mit einer Plattendichtung (113) befestigt sind, wobei die Dichtung aus porösem Material (148) und die Plattendichtung (113) unterschiedlich und voneinander getrennt sind.

4. Flüssigkeitssammelbaugruppe nach Anspruch 3, wobei die Dichtung aus porösem Material (148) und die Plattendichtung (113) verschiedene Arten von Befestigungen beinhalten.

5. Flüssigkeitssammelbaugruppe nach einem der vorhergehenden Ansprüche, wobei nur der untere Endbereich des mindestens einen porösen Materials (122) an der oberen Platte (108) und der unteren Platte (110) befestigt ist.

6. Flüssigkeitssammelbaugruppe nach einem der vorhergehenden Ansprüche, wobei der obere Endbereich (140) des mindestens einen porösen Materials (122) nicht an der oberen Platte (108) und der unteren Platte (110) befestigt ist.

7. Flüssigkeitssammelbaugruppe nach einem der vorhergehenden Ansprüche, wobei im Wesentlichen alle Ränder des mindestens einen porösen Materials (122) fest an der oberen Platte (108) und der unteren Platte (110) befestigt sind.

8. Flüssigkeitssammelbaugruppe nach einem der vorhergehenden Ansprüche, wobei die Dichtung aus porösem Material (148) konfiguriert ist, um den Kontakt zwischen dem mindestens einen porösen Material (122) und dem Flüssigkeitsauslass (118) oder einem Einlass der Leitung aufrechtzuerhalten.

9. Flüssigkeitssammelbaugruppe nach einem der vorhergehenden Ansprüche, wobei die Dichtung aus porösem Material (148) einen Klebstoff beinhaltet, der das mindestens eine poröse Material (122) an mindestens einer der oberen Platte (108) oder der unteren Platte (110) befestigt.

10. Flüssigkeitssammelbaugruppe nach einem der vorhergehenden Ansprüche, wobei die Dichtung aus porösem Material eine Schweißnaht beinhaltet, die das mindestens eine poröse Material (122) an mindestens einer der oberen Platte (108) oder der unteren Platte (110) befestigt.

11. Flüssigkeitssammelbaugruppe nach einem der vorstehenden Ansprüche, wobei die Dichtung aus porösem Material (148) eine Naht beinhaltet, die das mindestens eine poröse Material (122) an der oberen Platte (108) oder der unteren Platte (110) befestigt.

12. Flüssigkeitssammelbaugruppe nach einem der vorhergehenden Ansprüche, wobei mindestens der untere Endbereich des mindestens einen porösen Materials (122) an der oberen Platte (108) unter Verwendung einer ersten Dichtung aus porösem Material und an der unteren Platte (110) unter Verwendung einer zweiten Dichtung aus porösem Material befestigt ist, und wobei sich die erste Dichtung aus porösem Material von der zweiten Dichtung aus porösem Material unterscheidet.

13. Flüssigkeitssammelsystem (660), umfassend:
die Flüssigkeitssammelbaugruppe nach einem der Ansprüche 1-12;
einen Flüssigkeitsspeicherbehälter (662); und
eine Vakuumquelle (664);
wobei die Kammer der Flüssigkeitssammelbaugruppe, der Flüssigkeitsspeicherbehälter (662) und die Vakuumquelle (664) in Fluidkommunikation miteinander stehen, so dass, wenn eine oder mehrere Körperflüssigkeiten in der Kammer vorhanden sind, ein von der Vakuumquelle (664) an die Kammer der Flüssigkeitssammelbaugruppe bereitgestellter Sog die eine oder die mehreren Körperflüssigkeiten aus der Kammer entfernt und die Körperflüssigkeiten in dem Flüssigkeitsspeicherbehälter (662) ablagert.

14. Verfahren zum Bilden einer Flüssigkeitssammelbaugruppe nach einem der Ansprüche 1-12, wobei das Verfahren Folgendes umfasst:
Bereitstellen mindestens eines porösen Materials (122), das in einer Kammer (112) angeordnet ist, die durch eine flüssigkeitsundurchlässige Barriere (106) definiert ist, wobei die flüssigkeitsundurchlässige Barriere (106) mindestens die Kammer (112), mindestens eine Öffnung und einen Flüssigkeitsauslass (118) definiert, wobei die flüssigkeitsundurchlässige Barriere (106) eine obere Platte (108) und eine der oberen Platte (108) gegenüberliegende untere Platte (110) beinhaltet, wobei das mindestens eine poröse Material (122) einen oberen Endbereich und einen unteren Endbereich beinhaltet; und
festes Befestigen des mindestens einen porösen Materials an mindestens einer der oberen Platte (108) oder der unteren Platte (110) mit einer Dichtung aus porösem Material (148).

15. Verfahren nach Anspruch 14, wobei das Bereitstellen mindestens eines porösen Materials (122), das in der Kammer angeordnet ist, das Bereitstellen des mindestens einen porösen Materials (122) beinhaltet, das zwischen der oberen Platte (108) und der unteren Platte (110) positioniert ist, wobei die obere Platte (108) und die untere Platte (110) nicht aneinander befestigt sind; und
wobei das feste Befestigen des mindestens einen porösen Materials (122) an mindestens einer der oberen Platte (108) oder der unteren Platte (110) mit der Dichtung aus porösem Material (148) das feste Befestigen von Rändern der oberen Platte (108) an der unteren Platte (110) und das gleichzeitige Befestigen des mindestens einen porösen Materials (122) an der oberen Platte (108) und der unteren Platte (110) beinhaltet.

16. Verfahren nach Anspruch 14, wobei:
das Bereitstellen mindestens eines porösen Materials (122), das in der Kammer (112) angeordnet ist, das Bereitstellen des mindestens einen porösen Materials (122) beinhaltet, das an einer von der oberen Platte (108) oder der unteren Platte (110) befestigt ist; und
das feste Befestigen des mindestens einen porösen Materials (122) an mindestens einer von der oberen Platte (108) oder der unteren Platte (110) das feste Befestigen des mindestens einen porösen Materials (122) an der anderen von der oberen Platte (108) oder der unteren Platte (110) beinhaltet.

## Revendications

1. Ensemble de collecte des fluides (100), comprenant :
une barrière imperméable aux fluides (106) définissant au moins une chambre (112), au moins une ouverture et une sortie de fluide (118), la barrière imperméable aux fluides (106) comprenant un panneau supérieur (108) et un panneau inférieur (110) situé à l'opposé du panneau supérieur (108) ;
au moins un matériau poreux (122) disposé dans la chambre (112), l'au moins un matériau poreux (122) comprenant une région d'extrémité supérieure (140) et une région d'extrémité inférieure (142) ;
**caractérisé en ce que**
les bords d'au moins la région d'extrémité inférieure (142) de l'au moins un matériau poreux (122) sont fixés de manière rigide au panneau supérieur (108) et au panneau inférieur (110) à l'aide d'un joint en matériau poreux (148).

2. Ensemble de collecte des fluides selon la revendication 1, dans lequel le panneau supérieur (108) est distinct du panneau inférieur (110), et dans lequel les bords du panneau supérieur (108) sont fixés aux bords correspondants du panneau inférieur (110) à l'aide d'un joint de panneau (113), le joint en matériau poreux (148) et le joint de panneau (113) étant identiques.

3. Ensemble de collecte des fluides selon la revendication 1, dans lequel le panneau supérieur (108) est distinct du panneau inférieur (110), et dans lequel les bords du panneau supérieur (108) sont fixés aux bords correspondants du panneau inférieur (110) à l'aide d'un joint de panneau (113), le joint en matériau poreux (148) et le joint de panneau (113) étant distincts et séparés l'un de l'autre.

4. Ensemble de collecte des fluides selon la revendication 3, dans lequel le joint en matériau poreux (148) et le joint de panneau (113) comportent des types de fixation différents.

5. Ensemble de collecte des fluides selon l'une quelconque des revendications précédentes, dans lequel seule la région d'extrémité inférieure de l'au moins un matériau poreux (122) est fixée au panneau supérieur (108) et au panneau inférieur (110).

6. Ensemble de collecte des fluides selon l'une quelconque des revendications précédentes, dans lequel la région d'extrémité supérieure (140) de l'au moins un matériau poreux (122) n'est pas fixée au panneau supérieur (108) ni au panneau inférieur (110).

7. Ensemble de collecte des fluides selon l'une quelconque des revendications précédentes, dans lequel pratiquement tous les bords de l'au moins un matériau poreux (122) sont fixés de manière rigide au panneau supérieur (108) et au panneau inférieur (110).

8. Ensemble de collecte des fluides selon l'une quelconque des revendications précédentes, dans lequel le joint en matériau poreux (148) est configuré pour maintenir le contact entre l'au moins un matériau poreux (122) et la sortie de fluide (118) ou une entrée du conduit.

9. Ensemble de collecte des fluides selon l'une quelconque des revendications précédentes, dans lequel le joint en matériau poreux (148) comprend un adhésif fixant l'au moins un matériau poreux (122) à au moins l'un parmi le panneau supérieur (108) et le panneau inférieur (110).

10. Ensemble de collecte des fluides selon l'une quelconque des revendications précédentes, dans lequel le joint en matériau poreux comprend une soudure fixant l'au moins un matériau poreux (122) à au moins l'un parmi le panneau supérieur (108) et le panneau inférieur (110).

11. Ensemble de collecte des fluides selon l'une quelconque des revendications précédentes, dans lequel le joint en matériau poreux (148) comprend une couture fixant l'au moins un matériau poreux (122) à au moins l'un parmi le panneau supérieur (108) et le panneau inférieur (110).

12. Ensemble de collecte des fluides selon l'une quelconque des revendications précédentes, dans lequel au moins la région d'extrémité inférieure de l'au moins un matériau poreux (122) est fixée au panneau supérieur (108) à l'aide d'un premier joint en matériau poreux et au panneau inférieur (110) à l'aide d'un second joint en matériau poreux, et dans lequel le premier joint en matériau poreux est différent du second joint en matériau poreux.

13. Système de collecte des fluides (660), comprenant :
l'ensemble de collecte des fluides selon l'une quelconque des revendications 1 à 12 ;
un récipient de stockage des fluides (662) ; et
une source de vide (664) ;
dans lequel la chambre de l'ensemble de collecte des fluides, le récipient de stockage des fluides (662), et la source de vide (664) sont en communication fluidique les uns avec les autres de sorte que, lorsqu'un ou plusieurs fluides corporels sont présents dans la chambre, une aspiration fournie par la source de vide (664) vers la chambre de l'ensemble de collecte des fluides extrait le ou les fluides corporels de la chambre et les dépose dans le récipient de stockage des fluides (662).

14. Procédé de fabrication d'un ensemble de collecte des fluides, selon l'une quelconque des revendications 1 à 12, le procédé comprenant :
la mise en place d'au moins un matériau poreux (122) disposé dans une chambre (112) délimitée par une barrière imperméable aux fluides (106), la barrière imperméable aux fluides (106) délimitant au moins la chambre (112), au moins une ouverture, et une sortie de fluide (118), la barrière imperméable aux fluides (106) comprenant un panneau supérieur (108) et un panneau inférieur (110) opposé au panneau supérieur (108), l'au moins un matériau poreux (122) comprenant une région d'extrémité supérieure et une région d'extrémité inférieure ; et
la fixation de manière rigide de l'au moins un matériau poreux à au moins l'un parmi le panneau supérieur (108) et le panneau inférieur (110) à l'aide d'un joint en matériau poreux (148).

15. Procédé selon la revendication 14, dans lequel la mise en place d'au moins un matériau poreux (122) disposé dans la chambre comprend la mise en place de l'au moins un matériau poreux (122) entre le panneau supérieur (108) et le panneau inférieur (110), le panneau supérieur (108) et le panneau inférieur (110) n'étant pas fixés l'un à l'autre ; et
dans lequel la fixation de l'au moins un matériau poreux (122) à au moins l'un parmi le panneau supérieur (108) et le panneau inférieur (110) à l'aide du joint en matériau poreux (148) comprend la fixation des bords du panneau supérieur (108) au panneau inférieur (110) et la fixation de l'au moins un matériau poreux (122) au panneau supérieur (108) et au panneau inférieur (110) simultanément.

16. Procédé selon la revendication 14, dans lequel :
la mise en place d'au moins un matériau poreux (122) disposé dans la chambre (112) comprend la mise en place de l'au moins un matériau poreux (122) fixé à l'un des panneaux supérieur (108) ou inférieur (110) ; et
la fixation de l'au moins un matériau poreux (122) à au moins l'un parmi le panneau supérieur (108) et le panneau inférieur (110) comprend la fixation de l'au moins un matériau poreux (122) à l'autre parmi le panneau supérieur (108) et le panneau inférieur (110).
